Europäisches Patentamt

European Patent Office

Office européen des brevets

(19)

(11) **EP 1 608 979 B1**

(12) **EUROPÄISCHE PATENTSCHRIFT**

(45) Veröffentlichungstag und Bekanntmachung des Hinweises auf die Patenterteilung:
**22.11.2006 Patentblatt 2006/47**

(21) Anmeldenummer: **04724247.4**

(22) Anmeldetag: **30.03.2004**

(51) Int Cl.:
*G01N 33/573* [(2006.01)] *G01N 33/543* [(2006.01)]

(86) Internationale Anmeldenummer:
**PCT/DE2004/000674**

(87) Internationale Veröffentlichungsnummer:
**WO 2004/087946 (14.10.2004 Gazette 2004/42)**

(54) **VERFAHREN ZUM NACHWEIS POSTTRANSLATIONALER MODIFIZIERUNGSAKTIVITÄTEN UND GERÄTESYSTEME ZUR REALISIERUNG DES VERFAHRENS**

METHOD FOR THE DETECTION OF POST-TRANSLATIONAL MODIFICATION ACTIVITIES AND DEVICE SYSTEM FOR CARRYING OUT SAID METHOD

PROCÉDÉ D'IDENTIFICATION D'ACTIVITÉS DE MODIFICATION POST-TRANSLATIONNELLES ET SYSTÈMES D'APPAREILS PERMETTANT LA MISE EN OEUVRE DUDIT PROCÉDÉ

(84) Benannte Vertragsstaaten:
**AT BE BG CH CY CZ DE DK EE ES FI FR GB GR HU IE IT LI LU MC NL PL PT RO SE SI SK TR**

(30) Priorität: **02.04.2003 DE 10314965**

(43) Veröffentlichungstag der Anmeldung:
**28.12.2005 Patentblatt 2005/52**

(73) Patentinhaber: **Bioscora GmbH**
**04103 Leipzig (DE)**

(72) Erfinder: **VASILETS, Larissa**
**06179 Teutschenthal (DE)**

(74) Vertreter: **Voigt, Wolf-Rüdiger et al**
**Patentanwalt,**
**Alter Markt 1-2**
**06108 Halle (DE)**

(56) Entgegenhaltungen:
WO-A-87/03095 WO-A-02/095058
DE-A- 10 051 252 US-A1- 2001 004 522
US-B1- 6 410 255

• MANN M ET AL: "Analysis of protein phosphorylation using mass spectrometry: deciphering the phosphoproteome" TRENDS IN BIOTECHNOLOGY, ELSEVIER PUBLICATIONS, CAMBRIDGE, GB, Bd. 20, Nr. 6, 1. Juni 2002 (2002-06-01), Seiten 261-268, XP004352765 ISSN: 0167-7799
• YAN J X ET AL: "Protein phosphorylation: technologies for the identification of phosphoamino acids" JOURNAL OF CHROMATOGRAPHY A, ELSEVIER SCIENCE, NL, Bd. 808, Nr. 1-2, 29. Mai 1998 (1998-05-29), Seiten 23-41, XP004122656 ISSN: 0021-9673

**Beschreibung**

Die Erfindung betrifft ein Verfahren zum qualitativen Nachweis posttranslationaler Modifizierungsaktivitäten, das heißt den Nachweis enzymatischer Aktivitäten, die durch Bildung spezifischer Gruppen, wie die Phosphat-Gruppe, die bereits synthetisierten Proteine modifizieren und deren Funktion verändern.

Die Ursache für die Entstehung gravierender Krankheiten, wie Krebs, Diabetes, Arthritis, Herz-Kreislauf-Erkrankungen, Bluthochdruck und Schlaganfälle sind veränderte. Protein-Aktivitäten. Die Erfindung stellt ein schnelles, hochsensibles und effizientes Verfahren zum Nachweis der verschiedenen Typen der posttranslationalen Aktivitäten dar. Mit dem erfindungsgemäßen Verfahren und den Gerätesystemen wird im Bereich der biologischen Multidetektionssysteme ein wesentlicher Beitrag zum Hochdurchsatz erforderlicher Tests geleistet.

Bevor zum Stand der Technik nähere Ausführungen erfolgen, soll einleitend festgestellt werden:

Systeme und Methoden zum schnellen Nachweis von Analyten mit biologischen Aktivitäten, insbesondere in kleinen flüssigen Proben, haben eine hohe medizinische und pharmazeutische Bedeutung sowie Bedeutung im Umweltschutz. Eine der umfassendsten und bedeutenstn. Gruppen der zellulären Aktivitäten, die für die Pharmaka-Entwicklung besonders wichtig sind, sind die Aktivitäten, die in der posttranslationalen Modifizierung wirken. Die Folgen dieser Aktivitäten, die für jede lebende Zelle charakteristisch sind, sind die veränderten funktionellen Eigenschaften der modifizierten Eiweiße (Proteine). Die Hauptmechanismen der posttranslationalen Modifizierung der Proteine oder Polypeptide sind Phosphorylierung, Methylierung, Prenylierung, Ubiquitinierung und Proteolyse. Verschiedene externe Bedingungen (Stimuli), wie die Anwesenheit von Wachstumsfaktoren oder die Entwicklung von pathologischen Zuständen, wie die Veränderungen im Zellzyklus und die Wirkung von Toxinen, können vorübergehend den posttranslationalen Zustand mehrerer intrazellulärer Komponenten verändern. Deshalb ist die schnelle Entwicklung von spezifischen und effektiven Hemmern oder Aktivatoren von bestimmten posttranslationalen Aktivitäten notwendig. Insofern ist die Entwicklung von entsprechenden Proben und Verfahren wichtig, die einen zuverlässigen und empfindlichen Nachweis dieser Aktivitäten in Multidetektionssystemen (Microarrays, Biochips) ermöglichen.

Als Beispiel einer posttranslationalen Modifizierung dient die Phosphorylierung-Dephosphorylierung der Proteine durch Kinasen und Phosphathasen. Die Kinasen modifizieren die Proteine durch Bindung einer Phosphatgruppe (Phosphorylierung) an Aminosäure Reste, überwiegend Serin, Threonin oder Tyrosin. Im Gegensatz dazu entfernen Phosphatasen diese Phosphatgruppen und kehren damit den Phosphorylierungseffekt um. Die Veränderungen im Phosphorylierungszustand der Proteine regulieren enzymatische Aktivitäten durch Lokalisierung und molekulare Wechselwirkungen zwischen Proteinen in den lebenden Zellen. Die allgemeine Balance von Kinasen- und Phosphatasen-Aktivitäten in einer. Zelle ist Grundlage des Proteinphosphorylierungszustandes zu jedem Zeitpunkt. Die Wirkung von Protein Kinasen und Phosphatasen gehört allgemein betrachtet zu den wichtigsten regulatorischen Mechanismen der Protein-Funktionen. Die neusten Erkenntnisse und Analysen von Krankheiten, die auf genetische Defekte von Protein Kinasen hinweisen, deuten auf über 400 spezifische Krankheitszustände hin, die mit veränderten Aktivitäten der Kinasen selbst in Verbindung zu sehen sind.

Da die abnormale Proteinphosphorylierung Ursache für die Entstehung gravierender Krankheiten wie Krebs, Diabetis, Arthritis, Herz-Kreislauf-Erkrankungen, Bluthochdruck und Schlaganfälle ist, sind für die pharmazeutische Industrie neue Verbindungen von Interesse, die in der Lage sind, Phosphorylierungsaktivitäten der Protein Kinasen zu hemmen.

Um neue effektive Pharmaka zu entwickeln, müssen große Mengen von Verbindungen, die mit Hilfe von kombinatorischer Chemie synthetisiert sind, getestet werden. Für diese Zwecke benötigt die Pharmaindustrie neue Technologien, die im "high-throughput" (Hochdurchsatz) Format erforderliche Tests ermöglichen.

Auch um die Wirkung bereits entwickelter Pharmaka zu verbessern ist es notwendig zu prüfen, inwieweit sie die Protein Kinasen-und Phosphatasen-Aktivitäten beeinflussen. Beispiel: Cyclosporin ist ein Immunsystemhemmer, ohne den keine Organtransplantationen möglich wären. Erst neuere Studien haben gezeigt, dass die Wirkungsmechanismen dieses Mittels durch die Hemmung von Protein Phosphatase PP2B funktioniert.

Zum Stand der Technik ist auszuführen.

Die an sich bekannte Biochip-Technologie ist ein sehr effizientes Verfahren, das die medizinische Diagnostik und die Entwicklung von Pharmaka revolutioniert. Mit Gen-Chips können z. B. komplette Transkriptionsmuster von Tumoren oder anderer Gewebe in einem Versuch erfasst werden. Die Entwicklung und Herstellung von Gen-Chips hat bereits ein fortgeschrittenes Stadium erreicht. Die veränderten Protein-Aktivitäten die, wie schon gesagt, die Ursache gravierender Krankheiten sind, können mit Hilfe von Gen-Chips nicht erforscht werden. Es ist daher notwendig, effiziente Protein-Chip-Technologien zu entwickeln. (Verfahren zur schnellen Auswertung veränderter Protein-Aktivitäten im Multidetektionsformat). Aufgrund der hohen Komplexität der Protein-Chip-Technologien, stehen diese Technologien gegenwärtig nur bedingt zur Verfügung.

Zum internationalen Stand der Technik:

Die aktuellen Nachweismethoden von Kinasen-Aktivtäten basieren typischerweise auf Messungen durch Einbau des radioaktiven Phosphats $^{32}$P in Proteinsubstrate. Bei Anwendung dieser Methoden muss man sehr hohe Mengen von Radioaktivität für die Zellen verwenden, um den gesamten intrazellulären Pool von ATP zu markieren und die Radioaktivitätsmarkierung des Zielproteins zu sichern. Um die relative Phosphorylierung des Zielproteins nachzuweisen, müssen nach der Inkubierung von Zellen mit Testsubstanzen die Zellen zerlegt und das Zielprotein gereinigt werden. Diese Methode benötigt große Mengen von Zellen, langen Inkubierungszeiten und vorsichtige Behandlungsverfahren, um falsche Phosphoryllierungs-Dephosphorylierungsergebnisse zu vermeiden. Außerdem benötigt ein solches Verfahren die Reinigung des Zielproteins. Da die Endphosphorylierung des Zielproteins sehr niedrig sein kann, hat dieses Verfahren einen schlechten Wirkungsgrad. Besonders schwerwiegend für Umwelt und Gesundheit ist der hohe Verbrauch von Radioaktivität, den diese Methode in den Hochdurchsatz-Versuchen ("high-throughput") notwendig macht.

Alternative Methoden zu Bestimmung von Kinase-Aktivitäten sind solche, die auf phosphorylierungsspezifischen Antikörpern basieren wie ELISA und Western Blots. Der Nachteil dieser Methode besteht darin, dass es schwierig und sehr kostenintensiv ist, die Antikörper zu produzieren und zwischen phosphoryliertem und dephosphoryliertem Zustand des Proteins zu unterscheiden.

Die Massenspektrometrie (MALDI und ESI) wurde bereits vor über 15 Jahren zur Bestimmung von Primästrukturen proteolysierter Protein-Fragmente angewandt. Theoretisch wäre die Massenauflösung dieser Methode für den Nachweis von Phosphorylierungen mit Massendifferenz 80 Da ausreichend. Allerdings ist aufgrund der Instabilität der Phosphatbindung und deren schneller Abspaltung von Proteinresten während des Messverfahrens die Anwendung von MALDI-MS für den Phosphorylierungsnachweis nur bedingt möglich. Oft führt die massenspektrometrische Detektierung der Proteinphosphorylierung zu falschen negativen Ergebnissen. Darüber hinaus sind Massenspektrometer extrem kostenintensiv.

Aus dem Patent US 6,410,255 ist eine Methode bekannt, die es ermöglicht, Kinasen-Aktivitäten nachzuweisen. Diese Methode betrifft einen Sensor, der eine fluoreszierende Gruppe darstellt, die in einem Polypeptid zusammen mit einem Kinase-spezifischen Abschnitt und einem Protease-empfindlichen Abschnitt eingebaut ist. Die Modifizierung der Proteine führt zu veränderter Zugänglichkeit der Spaltungsstelle der Proteasen und Abspaltung der fluoreszierenden Gruppe. Diese wird mit Hilfe eines Fluoreszenzmikroskops detektiert. Zusätzlich zu Kinasen benötigt das Verfahren die Anwesenheit von Proteasen, zwischen deren eine Wechselwirkung möglicherweise zu Artefakten führen kann. Nachteilig sind die hoher Kosten der Floureszenzmikroskope und die Notwendigkeit einer Fluoreszenzmarkierung des Zielpeptides.

Abgesehen von den bisher erläuterten Nachteilen beim Nachweis von Aktivitäten der Kinasen und Phosphathasen kann zusammenfassend bis hierher festgestellt werden, dass nur begrenzte Möglichkeiten von schnellen Nachweisen dieser Aktivitäten in miniaturisierten Probevolumen mit Tausenden von Komponenten bestehen.

Die WO 87/03095 beschreibt einen offenen Kapazitätssensor für die Messungen der Konzentrationen von Analyten. Dieses Verfahren benötigt die Immobilisierung der Moleküle auf der Oberfläche eines offenen Kondensators und ist nur für die Proteinbindung vor gesehen.

Nun soll noch kurz auf die DE 100 51 252 A1 mit dem Titel "Bio-Chip" eingegangen werden. Diese DE beschreibt ein Verfahren zum qualitativen und/oder quantitativen Nachweis eines Analyten, welcher polarisierende Moleküle, insbesondere in molekularer Form gelöste Biomoleküle, aufweist. Ein Sensor mit einer Vielzahl kleiner Messzellen (< 1 mm $\varnothing$), die jeweils einen Kondensator darstellen, weist eine bestimmte Kapazität auf, soweit sich in den Messzellen ein Substrat befindet. Wird zusätzliches Material in der Form der nachzuweisenden Moleküle auf das Substrat gebracht, so ändert sich die Kapazität des Kondensators. Die Kapazitätsänderung steht im Zusammenhang mit der Konzentration des Analyten. Probleme bei der Gewinnung genauer Messdaten werden beim Einbringen der bioaktiven Substanzen in die Messzellen gesehen. Weiterhin ist ein feinmechanisches System mit Federkontakten für das Auswerten mit entsprechender Messelektronik mitunter auch der Anlass für Messfehler. Eine rationelle Möglichkeit von schnellen Nachweisen ist nicht möglich. Diese Lösung gemäß DE 100 51 252 A1 stellt ein Bio-Chip für die Messungen von Proteinbindungen dar; der Nachweis der posttranslationalen Modifizierungsaktivitäten ist nicht möglich.

Es ist daher die Aufgabe der Erfindung, ein automatisiertes Verfahren zur Bestimmung posttranslationaler Aktivitäten vorzuschlagen, welches hochsensibel und unkompliziert funktioniert und virtuell für alle Kinase- und Phosphathase - Aktivitäten anwendbar ist. Es sollen keine Farbstoffe, keine fluoreszierenden oder radioaktiven Substanzen verwendet werden.

Durch schnelle Nachweise der posttranslationalen Aktivitäten von Tausenden von Komponenten soll ein wesentlicher Beitrag auf den Gebieten der Forschung und Entwicklung von Pharmaka, der Grundlagenforschung, der Umwelttechnologie und molekularer medizinischer Diagnostik geleistet werden. Mit den entsprechenden Gerätesystemen erfolgt die Realisierung des Verfahrens.

Erfindungsgemäß wird die Aufgabe wie folgt gelöst, wobei hinsichtlich der grundlegenden Gedanken auf Patentanspruch 1 verwiesen wird. Die weitere Ausgestaltung der Erfindung ergibt sich aus den Patentansprüchen 2 bis 7.

Zur Darlegung der Erfindung sind weitere Erläuterungen erforderlich.

Die technische Lösung ermöglicht den schnellen und effektiven Nachweis der posttranslationalen Aktivitäten in flüssigen Proben mit Analyten bzw. Enzymen, die auf Veränderungen der physikalisch-chemischen Eigenschaften der jeweils verwendeten Sensoren basieren. Die flüssigen Proben sind die Proben aus Zellen oder die Proben mit Test-Substanzen, denen Enzyme hinzugegeben werden.

Die für den Nachweis der posttranslationalen Modifizierungsaktivitäten entwickelten Sensoren bestehen aus synthetisierten Proteinfragmenten bzw. Peptiden, die einen Erkennungsbereich für ProteinKinasen oder andere Enzyme enthalten. Auf die schematische Darstellung derartiger Sensoren wird im Zusammenhang mit den Erläuterungen zu den Ausführungsbeispielen eingegangen. Der Erkennungsbereich befindet sich zwischen zwei Gruppen der Aminosäuren Teilkette 1 und Teilkette 2, die die Anzahl von Aminosäuren 0 bis n haben und zusam-men mit dem Erkennungsbereich eine Reihe von geladenen Resten besitzen (siehe noch folgende Tabelle 1). Der Sensor ist derart konstruiert, dass er eine 3-dimensionale (3-D) Struktur mit spezifischer Verteilung des molekularen elektrostatischen Potentials und ein molekulares Dipolmoment $\mu$ besitzt. Als Folge der o. g. posttranslationalen Aktivitäten wird ein Modifikationsrest innerhalb des Erkennungsbereiches umgewandelt. Dementsprechend wird die elektrostatische Potentialverteilung verändert und es ergibt sich das Dipolmoment $\mu^*$ des Sensors. Diese Veränderungen des Dipolmoments des Sensors werden mit Hilfe elektrischer oder optischer Methoden, wie das in den Ausführungsbeispielen noch erläutert wird, nachgewiesen.

Die folgende Tabelle 1 zeigt Beispiele von Sensoren, die zum Nachweis von Protein Kinase A (PKA), Protein Phosphatase 2B (PP2B), Tyrosin Kinase (TK), Tyrosin Phosphatase (TP) und Protein Kinase C (PKC) Aktivitäten verwendet werden können.

Tabelle 1: Primär Strukturen der Sensoren (Fettdruck zeigt Modifikationsreste, Kursiv druck zeigt den Erkennungsbereich).

| Teilkette 1 | Erkennungsbereich | Teilkette 2 | Aktivität | Sensor Nr. |
|---|---|---|---|---|
| ELDVPIPGRFD | *RRVS* | VAAD | PKA | S1 |
| ELDVPIPGRFD | *RRVpS* | VAAD | PP2B | S2 |
| EI | *YETDYY* | D | TK | S3 |
| EI | *pYETDpYpY* | D | TP | S4 |
| EPEAVAEHG | *DKKS* | KKAKKER | PKC | S5 |

Die Entwicklung des Sensors mit gewünschter 3D-Struktur erfolgt durch molekulare Modellierung mit Hilfe von Bioinformatik-Methoden (z.B. Search in Swiss-Prot und PDB-Datenbanken, Optimierung der Struktur mit MOE oder SYBYL, Berechnung des molekularen elektrostatischen Potentials und des Dipolmoments der unmodifizierten und modifizierten Polypeptide). Hierzu wird verwiesen auf:

Brandt, W., Anders, A. and Vasilets, L. A. (2002) Predicted alterations in tertiary structure of the N terminus of the Na$^+$/K$^+$-ATPase $\alpha$ subunit caused by acidic replacement or PKCmediated phosphorylation of Ser-23. Cell. Biochem. Biophys. 37:83-95.

Beispiel: Das Dipolmoment des unphosphorylierten PKC-Sensors 5 (S5) lt. Tabelle 1 beträgt ca. 203 D, wobei die Phosphorylierung des Serins zur Orientierungsveränderung und Verminderung des Dipolmoments des Sensors auf 144 D führt.

Zusammenfassend ist bis hierher festzustellen:

Die Erfindung basiert auf dem Nachweis der posttranslationalen Aktivitäten in einer flüssigen Probe auf Veränderungen physikalisch-chemischer Eigenschaften des Sensors ohne Markierung des Zielpeptides. Als Beispiel der experimentellen Verfahren zur Detektierung der Veränderungen des Dipolmoments des Sensors können die Messungen der Veränderungen der Dielektrizitätskonstante (Permitivität), Relaxationsströme, Brechzahl, Dichte oder Intensität des polarisierten Lichtes genannt werden.

Die Erfindung soll nunmehr anhand von Ausführungsbeispielen mit ergänzenden Hinweisen erläutert werden.

Die Figuren zeigen

1 - Schematische Darstellung eines Sensors

EP 1 608 979 B1

2 - Schematische Darstellung eines Gerätes zum Nachweis der posttranslationalen Aktivität mit Hilfe von optischen Messungen

3 - Nachweis der Peptidmodifizierung durch Messungen der relativen Dielektrizitätskonstante ε

4 - Nachweis posttranslationaler Modifizierungsaktivitäten mit Hilfe differenzieller Messungen

5 - Nachweis der Peptidmodifizierung durch Messungen der Frequenzverschiebung eines Oszillators

In der Figur 1 zeigen die Bezugzeichen:

1 - Reihe der Aminosäuren (Teilkette 1)
2 - Reihe der Aminosäuren (Teilkette 2)
3 - Verbindung
4 - Bindungsstelle
5 - Festkörper
X - Modifizierungsreste

In der Figur 2 zeigen die Bezugzeichen

6 - Lichtquelle
7 - Polarisator
8 - Messzelle
9 - Lichtanalysator
10 - Lichtdetektor
P - polarisiertes Licht

In Figur 3 A zeigt das Bezugzeichen

11 - Meßzelle

In Figur 4 A zeigen die Bezugzeichen

12 - Frequenzgenerator
13 - Messkondensator C1
14 - Messkondensator C2
15 - differenzieller Verstärker
16 - Wechselstrom/Gleichstrom-Wandler (AC/DC-Wandler)

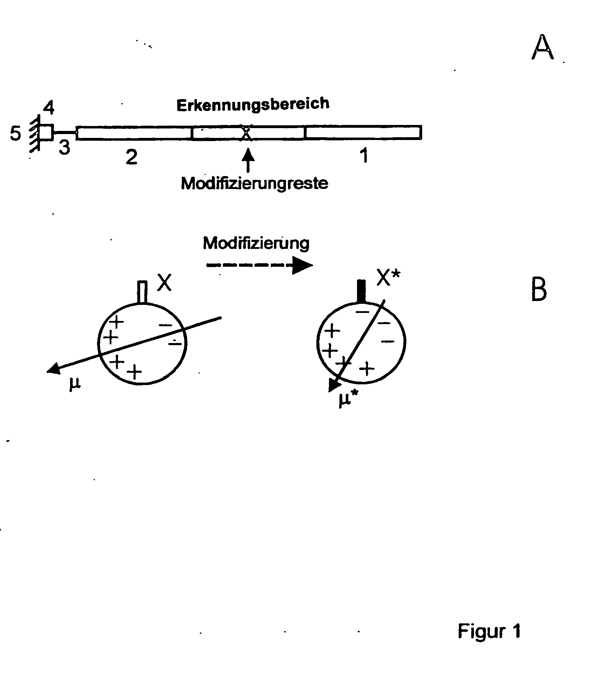

Figur 1 ist der Sensor, welcher erfindungsgemäß ein synthetisiertes Polypeptid darstellt, der ein Erkennungsbereich für ProteinKinasen oder andere Enzyme enthält. Figur 1A zeigt die schematische Darstellung eines solchen Sensors. Der Erkennungsbereich mit einem Modifizierungsrest oder mehreren Modifikationsresten X befindet sich zwischen zwei Gruppen der Aminosäuren (Teilkette 1 und Teilkette 2), die eine Reihe von geladenen Resten besitzen. Die Teilketten 1 und 2 sind zusammen mit dem Erkennungsbereich derart aufgebaut, dass sie zu einer 3-dimensionalen (3-D) Struktur mit spezifischer Verteilung des molekularen elektrostatischen Potentials führen. Somit besitzt der Sensor ein molekulares Dipolmoment $\mu$. Als Folge der o. g. posttranslationalen Aktivitäten wird ein Modifikationsrest X innerhalb des Erkennungsbereiches in X* umgewandelt. Somit werden die elektrostatische Potenzialverteilung und das Dipolmoment des Sensors $\mu^*$ verändert (Figur 1B). Diese Veränderungen der physikalisch-chemischen Eigenschaften des Sensors werden mit Hilfe elektrischer oder optischer Methoden (siehe unten) nachgewiesen.

In einer Variante der Erfindung kann der Sensor mit Hilfe einer flexiblen Verbindung 3 und einer Bindungsgruppe (His-tag) an einem mit Ni-NTA Harz-beschichteten Festkörper 5 befestigt befestigt werden (Figur 1), die eine Glasoberfläche, eine Kunststoffkugel ("bead") oder ein Dielektrikum darstellt.

In einer anderen Variante der Erfindung kann der Sensor direkt an der mit Dielektrikum beschichteten Festkörperoberfläche synthetisiert werden.

In einer weiteren Variante kann der Sensor in molekularer Form in Wasser gelöst sein.

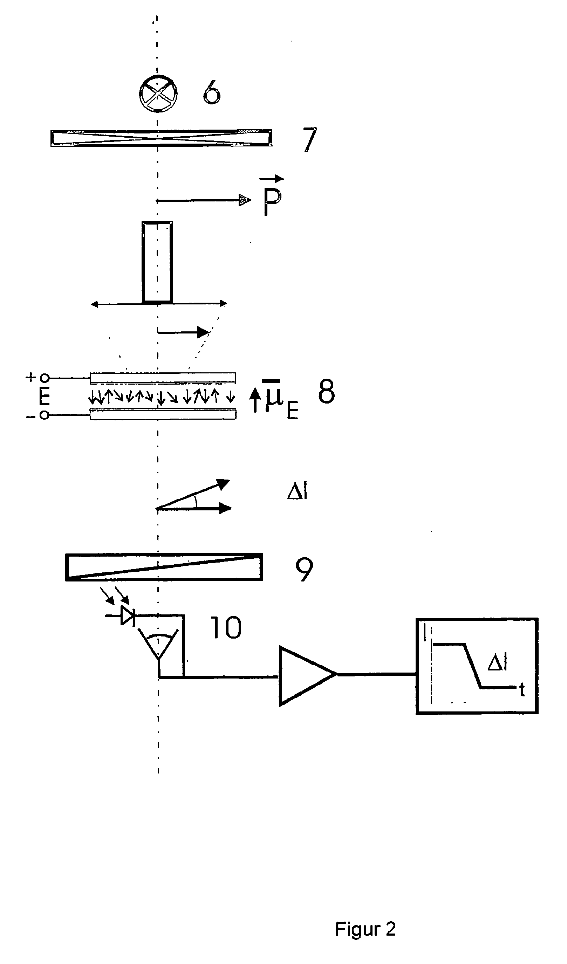

Figur 2 zeigt eine schematische Darstellung einer Methode zum Nachweis der posttranslationalen Aktivitäten als Folge der Veränderungen optischer Eigenschaften der Probe mit dem Sensor. Eine Detektionszelle (Messzelle 8) mit einer flüssigen Probe und einem Sensor befindet sich zwischen einer Quelle des polarisierten Lichts P und einem Lichtanalysators 9. Zwei dünne Glasplatten sind von innen mit einer Goldschicht und einem Dielektrikum überzogen. Diese dienen als Kontaktelektroden. Die Intensitätsveränderungen des detektierten Lichts $\Delta I$ sind proportional dem $\cos^2\alpha$ ($\alpha$ ist der Rotationswinkel des polarisierten Lichts P):

$$\Delta I \sim \cos^2 \alpha \qquad\qquad (1)$$

In Abwesenheit der elektrischen Spannung sind die Dipolmoleküle des Sensors wegen thermischer Bewegungen zufällig orientiert. Das Feld E bewirkt eine partielle Ausrichtung der Dipole, denn es konkurriert mit der thermischen Bewegung der Moleküle, die nach Bolzmannstatistik eine Zufallsverteilung der Dipolorientierungen anstreben. Unter den üblichen Messbedingungen $\mu E \ll kT$ kann das mittlere Moment in Feldrichtung als:

$$\bar{\mu}_E/\mu \simeq \mu E / 3kT \qquad\qquad (2)$$

berechnet werden, wobei $\mu$ das Dipolmoment einzelner Moleküle, $\bar{\mu}_E$ - das scheinbare mittlere Dipolmoment in Feldrichtung, E- die Feldstärke, T- die absolute Temperatur und $k = 1{,}3807 \cdot 10^{23}$ JK$^{-1}$ ist. Da der Rotationsgrad des polarisierten Lichts proportional der optischen Aktivität einer Probe mit Sensor, die andererseits proportional der Anzahl der Moleküle in Feldrichtung ist, ergibt das:

$$\Delta I \sim \cos^2 \alpha \sim N = \bar{\mu}_E/\mu = \mu E / 3kT \qquad\qquad (3)$$

Ändert sich das Molekulardipolmoment als Resultat einer posttranslationalen Aktivität, führt das zu Veränderungen der detektierten Lichtintensität.

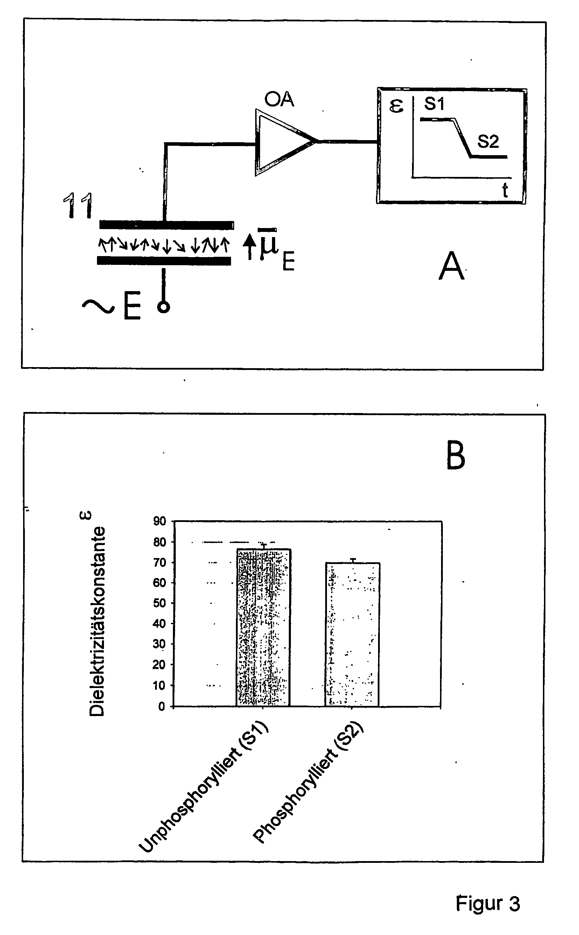

Gemäß einer anderen Variante zum Nachweis der posttranslationalen Aktivitäten wird die Dielektrizitätskonstante (Permitivität) einer flüssigen Probe mit einem Sensor gemessen. Wie aus Figur 3 A ersichtlich, befindet sich die Probe in einer Detektionszelle (Messzelle 11), die als Kondensator dient. Durch Einbringen eines polarisierenden Dielektrikums zwischen die Platten eines Kondensators wird die elektrischen Feldstärke im Vakuum $E_o$ auf $P/\varepsilon_o$ reduziert:

$$E = E_o - P/\varepsilon_o \qquad\qquad (4)$$

wobei $E_o = E/\varepsilon$, und P - ist induzierte Polarisation des Dielektrikums mit einer Dielektrizitätskonstante $\varepsilon$.

Das ergibt:

$$\varepsilon = \varepsilon_o + P/E \qquad\qquad (5)$$

Da Polarisation die Bedeutung eines Dipolmoments pro Volumeneinheit hat, ist P somit proportional dem scheinbaren mittleren Dipolmoment $\bar{\mu}_E$, welches nach Gleichung (2) $\bar{\mu}_E = \mu^2 E/3kT$ ist, das ergibt für $\varepsilon$:

$$\varepsilon = \varepsilon_o + \mu^2/kT \qquad\qquad (6)$$

Wird ein Dipolmoment aufgrund einer posttranslationalen Modifikation geändert, führt dies zu Veränderungen der Dielektrizitätskonstante nach Gleichung (6).

Werden die Sensormoleküle (s) in Wasser (w) oder anderen Lösungsmitteln gelöst, muss die Molpolarisation von diesen berücksichtigt werden:

$$P = P_W \cdot x_W + P_S \cdot x_S \qquad\qquad (7)$$

wobei $x_s$ der Molenbruch von Sensor $x_s = n_s/(n_w + n_s)$ und $x_w$ des Molenbruch von Wasser $x_w = n_w/(n_w + n_s)$ ist.

Figur 3 B zeigt ein Beispiel der Veränderungen der Dielektrozitätskonstante für zwei synthetisierte Modellpeptide S1 und S2 (Tabelle 1).

Die in Wasser gelösten Polypeptide (S1) oder (S2) sind als Dielektrikum zwischen den Elektrodenplatten des Messkondensators (Messzelle 11), siehe Figur 3A, angebracht. Polypeptid ELDVPIPGRFD*RRVS*VAAD (S1) ist ein spezifisches Substrat für Protein Kinase A, die Phosphorylierung des Serins katalysiert. Die Dephoshorylierung des Serins des Polypeptids ELDVPIPGRFD*RRVpS*VAAD (S2) erfolgt durch Phosphathase PP2B. Die Dielektrizitätskonstante des unphosphorylierten Polypeptides (S1) $\varepsilon_3$ beträgt 76, wobei diese des phosphorylierten Polypeptides (S2) auf 70 reduziert ist. Somit ermöglichen diese Verfahren die Aktivitäten dieser Enzyme nachzuweisen.

Zum Nachweis der Peptidmodifizierung im Versuch durch die Messungen der relativen Dielektrizitätskonstante $\varepsilon$ ist noch zu ergänzen: Vier $\mu$l von Proben mit jeweils Polypeptid S1 (unphosphoryliert) oder S2 (phosphoryliert) (Tabelle 1), die in $H_2O$ in Konzentration 1,14 mM gelöst sind, werden unmittelbar zwischen die Elektrodenplatten eines Messkondensators eingetragen. Die Messungen der Dielektrizitätskonstante $\varepsilon$ erfolgen bei einer Temperatur t=23°C. Im vorliegenden Beispiel sollten die niedrigsten Probevolumen nicht unter 4 $\mu$l liegen. Die Proteinkonzentrationen müssen sich im Millimolarbereich befinden, das kann die Verwendung dieser Messverfahren für Biochips einschränken.

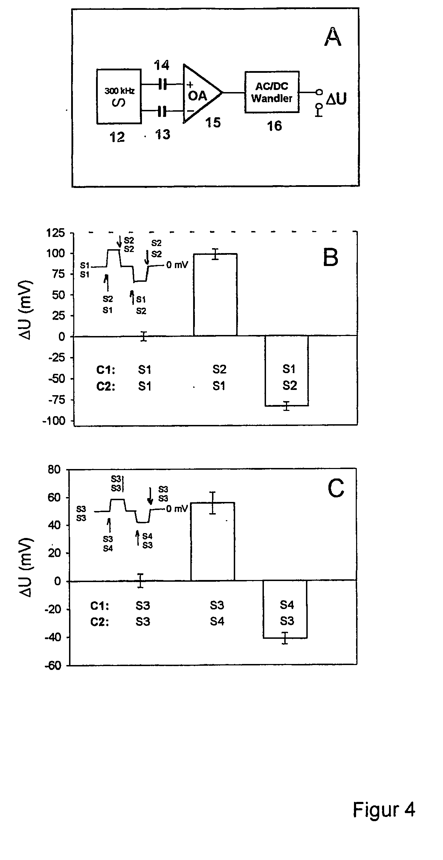

Um die Verwendung dieser Messverfahren für Biochips zu ermöglichen, wurden differenzielle Kapazitätsmessungen entwickelt, die das Probenvolumen unter 0,5 $\mu$l sowie die ProteinKonzentrationen auf $10^{-5}$ M reduzieren. Die Figuren 4 A und 4 B zeigen die Ergebnisse solcher differenziellen Messungen. Besonders vorteilhaft ist die hohe Zeitauflösung dieser elektrischen Messungen, die im ms-Bereich liegt und hauptsächlich von der Schnelligkeit des Probeeintrags abhängig ist. Somit ermöglichen diese Verfahren nicht nur Modifizierungsaktivitäten in kleinen flüssigen Proben nachzuweisen, sondern auch deren Kinetik zu registrieren.

Bezüglich des Aufbaus des Gerätes für differenzielle Messungen wird auf Figur 4 A verwiesen. Das Messgerät besteht aus dem Frequenzgenerator 12, den Messkondensatoren 13, 14, die in einer Chip-Platte integriert sind, dem Verstärker 15 und dem Wechselstrom/Gleichstrom-Wandler (AC/DC-Wandler) 16. Die Amplitude des Ausgangssignals, die proportional zur Differenz der Kapazitäten der Messkondensatoren 13 und 14 ist, wird in eine Gleichspannungsdifferenz mit Hilfe von AC/DC-Wandler $\Delta$U umgewandelt. Diese wurde mit Hilfe eines Schreibers registriert (s. Figur 4B und 4C) oder mit Hilfe eines Analog / Digitalwandlers für eine weitere digitale Auswertung umgewandelt.

Figur 4 B zeigt die differenziellen Messungen mit Sensoren für Protein Kinase A Aktivität: ELDVPIPGRFD*RRVS*VAAD (S1) und Phosphathase PP2B-Aktivität: ELDVPIPGRFD*RRVpS*VAAD (S2).

Figur 4 C zeigt die differentiellen Messungen mit den Sensoren für Tyrosin Kinase-Aktivität EI*YETD YY*D (S3) und für Tyrosin Phosphatase-Aktivität EI*pYETDpYpY*D (S4). Alle Sensoren sind in $H_2O$ bei einer Konzentration von 20 $\mu$M gelöst und entweder im Messkondensator C1 13 oder im C2 14 aufgetragen (siehe Beschriftung). Die Probenvolumen betragen 0,5 $\mu$l. Alle Messungen sind bei Raumtemperatur durchgeführt.

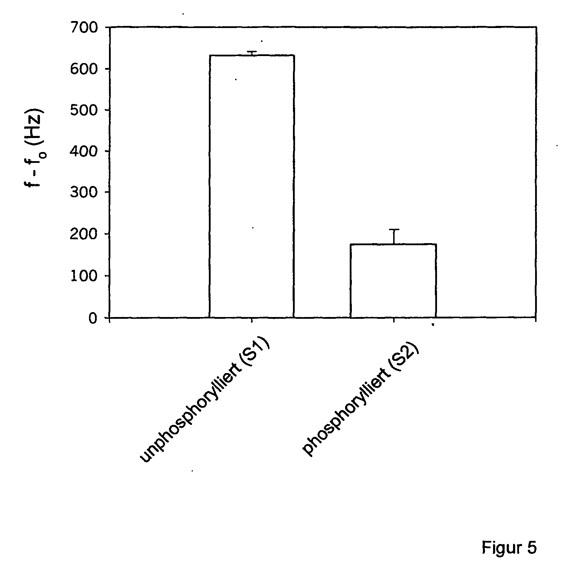

Gemäß einer weiteren Variante der Verfahren zum Nachweis der posttranslationalen Aktivitäten sind die in Wasser gelösten Polypeptide (S1) oder (S2) als Substrate in einer Messzelle angebracht, welche die Induktivität L und Kapazität C besitzt und als Oszillator dient (Figur 5). Die Frequenz $\omega$ des Oszillators lässt sich durch $\omega = 1/LC$ bestimmen, die mit hoher Genauigkeit ($10^{-5}$) gemessen sein kann. Es ist eine deutliche Verminderung der Frequenz zu sehen, wenn die Probe anstatt eines unphosphorylierten Polypeptids S1 ein phosphoryliertes Polypeptid S2 beinhaltet (Figur 5).

Zum Nachweis der Peptidmodifizierung durch Messungen der Frequenzverschiebung eines Oszillators wurde im Versuch wie folgt vorgegangen:

Ein Röhrchen mit 150 $\mu$l von Proben mit unphosphorylierten Peptid S1 oder phoshorylierten Peptid S2 (Konzentration 1,14 mM in $H_2O$) ist als Kern in einer Induktionsspule eines Oszillators platziert. Die Frequenz f ist bei Temperatur t = 22 °C gemessen, $f_o$ = 5342,9 kHz.

Die Peptidmodifizierung lässt sich auch durch Messungen des Brechungsindex ermitteln. Nach der Maxwell'schen Theorie des Magnetismus besteht zwischen der Dielektrizitätszahl und bei gleicher Frequenz gemessenen Brechungsindex n die Beziehung:

$\varepsilon_r = \varepsilon/\varepsilon_o = n^2$. Das ermöglicht die durch posttranslationalen Aktivitäten induzierte Veränderungen des molekularen Dipolmoments durch Veränderungen des Brechungsindex:

$$n = (1 + \mu^2/kT \; \varepsilon_o)^{1/2} \qquad\qquad (8)$$

zu detektieren.

Da die flüssigen Proben aus den Zellen oder Organismus sehr kompliziert sind und viele Proteine und kleinere polarisierende Moleküle besitzen, führt das zur großen Variabilität der o.g. physikalisch-chemischen Eigenschaften solcher Proben. Um die posttranslationalen Aktivitäten in komplizierten flüssigen Proben nachzuweisen, ist es wichtig, differenzielle Messungen durchzuführen. Dielektrizitätskonstante, Brechungsindex oder Lichtintensität können z.B. als Differenz zwischen Proben mit und ohne Sensor oder vor und nach einer Inkubierung mit einem Sensor detektiert werden.

Abschließend ist zusammenzufassen:

Das o. g. Verfahren zum Nachweis posttranslationaler Modifizierungsaktivitäten ermöglicht den Nachweis der Protein Kinasen- und Phosphathasen-Aktivitäten unter Verwendung der vorher erläuterten Gerätesysteme in einer Lösung mit einem losen oder befestigten Sensor bzw. Proteinfragment.

Durch differentielle Kapazitätsmessungen der posttranslationalen Modifizierungsaktivitäten, die in modifiziertem und nichtmodifiziertem Zustand unterschiedliche molekulare Dipolmomente besitzen, lassen sich die Probenvolumen unter 0,5 $\mu$l sowie die Sensor-Peptidkonzentrationen auf $10^{-5}$ M reduzieren. Somit ermöglichen diese Verfahren die Bestimmung mehrerer Modifizierungsaktivitäten in miniaturisierten Probenvolumen und eine Registrierung deren Kinetik.

Die entwickelte Elektronik für differenzielle Kapazitätsmessungen ermöglicht eine effektive unkomplizierte, schnelle und kostengünstige Detektion posttranslationaler Modifizierungsaktivitäten.

Der Anwendungsbereich der Erfindung erstreckt sich auf die Pharma-Industrie, (große und mittlere Pharma-Unternehmen), medizinische Diagnostik und Grundlagenforschung und auf Biotechnologie-Unternehmen mit der Zielstellung:

•  Verbesserung und Optimierung von Hochdurchsatz-Testreihen für neuesynthetisierte und bereits vorhandene Verbindungen
•  Untersuchung deren hemmender Wirkung auf Protein Kinasen- und Phosphathasen- Aktivitäten in vorklinischen Studien.
•  **Medizinische Diagnostik.**

Als Besonderheiten des erfindungsgemäßen Verfahrens sind hervorzuheben:

•  Die Möglichkeit der Kinetikmessungen mehrerer posttranslationaler Modifizierungsaktivitäten
•  Voraussetzungen zur Messung sehr instabiler Modifizierungen wie Phosphorylierung der Histamin und Arginin Aminosäure-Reste wurden geschaffen.

**Patentansprüche**

1.  Verfahren zum Nachweis posttranslationaler Modifizierungsaktivitäten, **dadurch gekennzeichnet, dass** im Sinne eines Sensors Proteinfragmente bzw. Polypeptide hergestellt werden, welche aus Aminosäuren zweier Teilketten 1 und 2 die eine Reihe von geladenen Resten besitzen, und einem Erkennungsbereich, welcher Modifizierungsreste (X) beinhaltet und eine molekulare elektrostatische Potentialverteilung aufweist, bestehen wobei die elektrostatische Potentialverteilung als Dipolmoment gemessen wird, dem Sensor die Enzyme hinzugegeben werden, eine erneute Messung des Dipolmoments erfolgt und eine Veränderung des elektrostatischen Potentials und damit des Dipolmoments ein Nachweis der posttranslationalen Modifizierungsaktivitäten darstellt.

2.  Verfahren nach Anspruch 1, **dadurch gekennzeichnet, dass** die Aminosäuren der zwei Teilketten 1 und 2 eine Anzahl von 0 bis n haben und zusammen mit dem Erkennungsbereich eine Reihe von geladenen Resten besitzen.

3.  Verfahren nach Anspruch 1, **dadurch gekennzeichnet, dass** der Erkennungsbereich mit dem Modifizierungsres den Modifizierungsresten (X) eine Erkennungsgruppe darstellt und die Umwandlung des Modifizierungsrestes der Modifizierungsreste nur durch spezifische Protein Kinase, Phosphathase oder andere Enzyme ermöglicht.

4.  Verfahren nach Anspruch 1, **dadurch gekennzeichnet, dass** die Protein-fragmente oder Polypeptide (Teilketten 1, 2 zusammen mit dem Erkennungsbereich und dem Modifizierungsrest den Modifizierungsresten (X)) eine 3D-Struktur mit herstellerseitig vorgegebener Verteilung des molekularen elektrostatischen Potentials und des molekularen Dipolmoments aufweisen.

5.  Verfahren nach Anspruch 1 bis 4, **dadurch gekennzeichnet, dass** die synthetisierten Proteinfragmente entweder in einer Lösung gelöst sind oder an einem Festkörper (5) platziert sind.

6.  Verfahren nach Anspruch 1, **dadurch gekennzeichnet, dass** eine Veränderung des molekularen Dipolmoments des Proteinfragments als Folge der posttranslationalen Modifizierungsaktivitäten gemessen wird, indem das Dipolmoment in eine andere physikalische Maßeinheit umgerechnet und als solche ausgewiesen wird und das Dipolmoment in der veränderten Größe der anderen physikalischen Maßeinheit gemessen bzw. aufgezeichnet wird.

7.  Verfahren nach Anspruch 1 und 6, **dadurch gekennzeichnet, dass** auf der Basis einer differentiellen Kapazitätsmessung die posttranslationalen Modifizierungsaktivitäten ermittelt werden, indem die Veränderung als die Veränderung einer Ausgangsspannung $\Delta U$ eines Verstärkers gemessen bzw. aufgezeichnet wird.

**Claims**

1.  Method for the detection of posttranslational modification activities, **characterised in that** protein fragments or polypeptides are utilized as sensors created from amino acid residues in form of the two part chains (moieties) 1 & 2 which possess a series of charged residues, and a recognition site containing modification residues X and displaying a molecular electrostatic potential distribution, whereby the electrostatic potential distribution is measured as a dipole moment, enzymes are added to the sensor, the dipole moment is measured for a second time and a change in the electrostatic potential and therefore dipole moment represents the detection of posttranslational modification activities.

**2.** Method according to claim 1 **characterised in that** amino acid residues of the two moieties 1 & 2 are present in a quantity from 0 to n and, in combination with the recognition site, possess a series of charged residues.

**3.** Method according to claim 1 **characterised in that** the recognition site in combination with the modification residue (s) (X) represents a recognition motif only permitting the conversion of the modification residue(s) through the specific protein kinase or phosphatase or other enzymes.

**4.** Method according to claim 1 **characterised in that** the protein fragments or polypeptides (as moieties 1 & 2 in combination with the recognition site and modification residue(s) (X)) display a three-dimensional structure with a distribution of molecular electrostatic potential and molecular dipole moment as prescribed by the manufacturer.

**5.** Method according to claims 1 to 4 **characterised in that** the synthesized protein fragments are either dissolved in a solution or attached to a solid body (5).

**6.** Method according to claim 1 **characterised in that** a change in the molecular dipole moment of the protein fragment as a consequence of posttranslational modification activities is measured by the conversion of the dipole moment into a different physical measurement unit and is indicated as such, and in which the dipole moment in the altered dimensions of the different physical measurement unit is measured and/or recorded.

**7.** Method according to claims 1 and 6 **characterised in that** the posttranslational modification activities are determined on the basis of a differential capacitance measurement through the measurement and/or recording of the changes of the output voltage $\Delta U$ of an amplifier or another detection device.

**Revendications**

**1.** Procédé pour la preuve des activités de modification par post-translation, **caractérisé par le fait que** dans le sens d'un capteur des fragments de protéines ou des polypeptides sont produits, lesquels sont constitués d'aminoacides de deux chaînes partielles 1 et 2 qui possèdent une série des restes chargés et d'un domaine de reconnaissance, lequel contient des restes de modification (X) et présente une répartition moléculaire électrostatique de potentiel, la répartition électrostatique de potentiel étant mesurée comme moment dipolaire, les enzymes étant donnés au capteur, une nouvelle mesure du moment dipolaire ayant lieu et une modification du potentiel électrostatique et ainsi du moment dipolaire représentant une preuve des activités de modification par post-translation.

**2.** Procédé d'après la revendication 1, **caractérisé par le fait que** les aminoacides des chaînes partielles 1 et 2 ont un nombre de 0 à n et qu'ils possèdent en commun avec le domaine de reconnaissance une série de restes chargés.

**3.** Procédé d'après la revendication 1, **caractérisé par le fait que** le domaine de reconnaissance représente avec le reste de modification/ les restes de modification (X) un groupe de reconnaissance et ne permet la transformation du reste de modification/ des restes de modification que par une protéine spécifique de kinase, phosphatase ou d'autres enzymes.

**4.** Procédé d'après la revendication 1, **caractérisé par le fait que** les fragments de protéine ou polypeptides (Chaînes partielles 1 et 2 en commun avec le domaine de reconnaissance et le reste de modification/ les restes de modification (X)) présentent une structure en 3-D avec une répartition du potentiel moléculaire électrostatique et du moment dipolaire moléculaire indiqués par le producteur.

**5.** Procédé d'après les revendications de 1 à 4, **caractérisé par le fait que** les fragments de protéine synthétisés sont dissous dans une solution ou sont placés sur un corps solide (5)

**6.** Procédé d'après la revendication 1, **caractérisé par le fait qu'**une modification du moment dipolaire moléculaire du fragment de protéine est mesurée comme conséquence des activités de modification par post-translation, du fait que le moment dipolaire est converti en une autre unité de mesure physique et identifié comme tel et que le moment dipolaire est mesuré et identifié dans la valeur modifiée de l'autre unité de mesure physique.

**7.** Procédé d'après les revendications 1 et 6, **caractérisé par le fait que** sur la base d'une mesure différentielle de capacité les activités de modification par post-translation sont déterminées, du fait que la modification est mesurée et enregistrée comme modification d'une tension de sortie $\Delta U$ d'un amplificateur.

Figur 1

Figur 2

Figur 3

Figur 4

Figur 5

SEQUENCE LISTING

<110> BIOSCORA GmbH
Vasilets, Larissa

<120> verfahren zum Nachweis posttranslationaler Modifizierungsaktivitäten und Gerätesyteme zur Realisierung des Verfahrens

<130>

<140> PCT/DE2004/000674
<141> 2004-03-30

<160> 5

<170> PatentIn version 3.1

<210> 1
<211> 19
<212> PRT
<213> Artificial

<400> 1

```
Glu Leu Asp Val Pro Ile Pro Gly Arg Phe Asp Arg Arg Val Ser Val
1               5               10                  15

Ala Ala Asp
```

<210> 2
<211> 19
<212> PRT
<213> Artificial

<220>
<221> MISC_FEATURE
<222> (15)..(15)
<223> X steht für phosphoryliertes Serin

<400> 2

```
Glu Leu Asp Val Pro Ile Pro Gly Arg Phe Asp Arg Arg Val Xaa Val
1               5               10                  15

Ala Ala Asp
```

<210> 3
<211> 9
<212> PRT
<213> Artificial

<400> 3

```
Glu Ile Tyr Glu Thr Asp Tyr Tyr Asp
1               5
```

<210> 4
<211> 9
<212> PRT
<213> Artificial

<220>
<221> MISC_FEATURE
<222> (3)..(8)
<223> X steht für phosphoryliertes Tyrosin

<400> 4

```
Glu Ile Xaa Glu Thr Asp Xaa Xaa Asp
1               5
```

<210> 5
<211> 20

<212> PRT
<213> Rattus norvegicus

<400> 5

Glu Pro Glu Ala Val Ala Glu His Gly Asp Lys Lys Ser Lys Lys Ala
1               5                       10                  15

Lys Lys Glu Arg
            20